# EUROPÄISCHE PATENTANMELDUNG

(11) **EP 0 611 750 A1**
(43) Veröffentlichungstag der Anmeldung: **24.08.1994**
(21) Anmeldenummer: 94101608.1
(22) Anmeldetag: 03.02.1994
(51) Int. Cl.: C07C 253/30, C07C 255/24, C07C 255/12

(54) **Verfahren zur Herstellung von Gemischen aus 3-Aminopropionitril und Ethylencyanhydrin**

(30) Priorität: 11.02.1993 DE 4304002
(71) Anmelder: BASF Aktiengesellschaft, D-67063 Ludwigshafen (DE)
(72) Erfinder: Merger, Franz, Dr., D-67227 Frankenthal (DE); Brudermueller, Martin, Dr., D-68165 Mannheim (DE); Priester, Claus-Ulrich, Dr., D-67065 Ludwigshafen (DE); Witzel, Tom, Dr., D-67069 Ludwigshafen (DE); Voit, Guido, Dr., D-69198 Schriesheim (DE); Harder, Wolfgang, Dr., D-69469 Weinheim (DE); Franz, Dieter, Dr., D-67069 Ludwigshafen (DE); Friedrich, Wolfgang, D-67346 Speyer (DE)

(57) **Zusammenfassung**

Verfahren zur Herstellung von Gemischen aus 3-Aminopropionitril der Formel I

H₂N-CH₂-CH₂-CN (I)

und Ethylencyanhydrin der Formel II

HO-CH₂-CH₂-CN (II)

indem man Bis-(2-cyanethyl)ether der Formel III

NC-CH₂-CH₂-O-CH₂-CH₂-CN (III)

bei Temperaturen von 50 bis 200°C und Drücken von 1 bis 500 bar mit Ammoniak umsetzt.

## Beschreibung

Die vorliegende Erfindung betrifft ein neues Verfahren zur Herstellung von Gemischen aus Aminopropionitril und Ethylencyanhydrin durch Umsetzung von Bis-(2-cyanethyl)-ether mit Ammoniak bei erhöhten Temperaturen.

Es ist bekannt, daß bei der Herstellung von 3-Aminopropionitril die Monoaddition von Acrylnitril an Ammoniak stets in erheblichem Maße auch von der Bildung des Bisadduktes Bis-(2-cyanethyl)amin begleitet ist.

Aus der US-A-2 401 429 ist bekannt, daß man nach 2 Tagen aus Acrylnitril und flüssigem Ammoniak bei Raumtemperatur neben 11 % 3-Aminopropionitril auch 76 % Bis-(2-cyanethyl)amin als Hauptprodukt isolieren kann. Bei 90°C setzt sich Acrylnitril unter Druck mit flüssigem Ammoniak zu 13 % 3-Aminopropionitril um (DE-A-598 185).

Es ist auch bekannt, daß sich der Zusatz von protischen Lösungsmitteln vorteilhaft auf die Addition von Ammoniak an Acrylnitril auswirkt. Jedoch auch bei einem Verhältnis Ammoniak/Acrylnitril/Wasser von 5 bis 15 : 1 : 5 bis 20 erhält man 3-Aminopropionitril neben Bis-(2-cyanethyl)amin nur in Ausbeuten von 57 bis 80 % (siehe z. B. US-A-3 935 256, DE-A-24 36 651, US-A-2 448 013, US-A-2 819 903).

Die kommerzielle Herstellung von Ethylencyanhydrin erfolgt bislang aus Ethylenoxid und Blausäure (US-A-2 653 162)

Die selektive Herstellung von Ethylencyanhydrin aus Acrylnitril/Wasser verläuft jedoch wie bei der Herstellung von 3-Aminopropionitril aus Acrylnitril/Ammoniak mit unbefriedigender Selektivität. In Gegenwart von Basen führt die Addition von Wasser an Acrylnitril neben der Bildung von Ethylencyanhydrin auch zum Bisaddukt Bis-(2-cyanethyl)-ether als Hauptprodukt (z. B. DE-A-11 89 975, US-A-2 816 130, JP 83-71444).

Die Reaktion verläuft zudem nur unter unvollständigem Umsatz des Acrylnitrils und führt zur Bildung von schwer abtrennbaren Nebenprodukten (Bildung von Amiden). Zur Isolierung von Ethylencyanhydrin müssen daher wäßrige Acrylnitrillösungen aufgearbeitet werden. Die Handhabung und die destillative Aufarbeitung dieser Acrylnitrillösungen sind (abgesehen von zusätzlichen Kosten, welche durch sie verursacht werden) insofern problematisch, als Acrylnitril toxisch ist und außerdem bei der Destillation zur Polymerisation neigt.

Zur Herstellung von Ethylencyanhydrin eignen sich daher besser Verfahren zur Spaltung von Bis-(2-cyanethyl)ether unter gleichzeitiger Bindung des dabei anfallenden Acrylnitrils.

Im Gegensatz zur Synthese von Ethylencyanhydrin ist die gezielte Synthese von Bis-(2-cyanethyl)ether durch Addition von einem Wassermolekül an zwei Moleküle Acrylnitril mit guten Ausbeuten beschrieben.

Aus der EP-A-352 504 ist beispielsweise die Umsetzung von Acrylnitril mit Wasser in Gegenwart von Mineralbasen oder quartären Stickstoffbasen bekannt.

JP-A 81 85 550 beschreibt die Spaltung des Ethers bei Temperaturen von 75 bis 200°C in Gegenwart von Basen wie Tetraethylammoniumacetat bzw. Alkalihydroxiden zu Ethylencyanhydrin und Acrylnitril. In diesem Verfahren sind die gesamten Probleme des Umlaufes von freiem Acrylnitril nicht gelöst.

Nach der DE-A-35 22 906 kann der Ether mit Methanolaten zu Ethylencyanhydrin und 3-Methoxypropionitril umgesetzt werden. Dieses Verfahren liefert zwar gute Ausbeuten, jedoch fallen dabei zwangsläufig große Mengen 3-Methoxypropionitril an, welches aber nur in beschränktem Umfang Verwendung findet.

Gleiches gilt für die im EP-A-352 504 beschriebene gemeinschaftliche Herstellung von Ethylencyanhydrin und 3-Dialkylaminopropionitrilen für 3-Dialkylaminopropionitrile.

Der vorliegenden Erfindung lag daher die Aufgabe zugrunde, den zuvor genannten Nachteilen abzuhelfen.

Demgemäß wurde ein neues Verfahren zur Herstellung von Gemischen aus 3-Aminopropionitril der Formel I

H₂N-CH₂-CH₂-CN (I)

und Ethylencyanhydrin der Formel II

HO-CH₂-CH₂-CN (II)

gefunden, welches dadurch gekennzeichnet ist, daß man Bis-(2-cyanethyl)ether der Formel III

NC-CH₂-CH₂-O-CH₂-CH₂-CN (III)

bei Temperaturen von 50 bis 200°C und Drücken von 1 bis 500 bar mit Ammoniak umsetzt.

Das vorliegende Verfahren läßt sich wie folgt durchführen:
Bis-(2-cyanethyl)ether III und Ammoniak können bei Temperaturen von 50 bis 200°C, bevorzugt 90 bis 170°C, besonders bevorzugt 100 bis 150°C und Drücken von 1 bis 500 bar, bevorzugt 20 bis 300 bar, besonders bevorzugt 100 bis 200 bar in einem geeigneten Reaktor durchgeführt werden.

Zur Durchführung der Spaltung kann sowohl die diskontinuierliche als auch die kontinuierliche Fahrweise gewählt werden. Bei der diskontinuierlichen Umsetzung ist das Arbeiten unter Atmosphärendruck zweckmäßig, dagegen wird beim kontinuierlichen Verfahren die Druckfahrweise bevorzugt. Bevorzugt wird die Umsetzung kontinuierlich unter erhöhtem Druck von 100 bis 200 bar.

Bei der kontinuierlichen Umsetzung z.B. im Rohrreaktor werden Verweilzeiten von 0,5 min bis 180 min, vorzugsweise 5 bis 60 min, bezogen auf das Reaktorvolumen eingehalten.

Die Spaltung von Bis-(2-cyanethyl)ether III zu Ethylencyanhydrin II und 3-Aminopropionitril I mit Ammoniak besteht aus zwei parallel ablaufenden Teilreaktionen. Zum einen die Spaltung des Bis-(2-cyanethyl)-ethers III in Ethylencyanhydrin II und Acrylnitril und zum anderen aus der Addition von Ammoniak an Acrylnitril.

Ammoniak und der Bis-(2-cyanethyl)ether III werden in der Regel im Molverhältnis von 1 : 1 bis 100 : 1, bevorzugt 10 : 1 bis 50 : 1, besonders bevorzugt 10 : 1 bis 30 : 1 eingesetzt. Ammoniak kann in flüssiger, wasserfreier Form oder als wäßrige Lösung verwendet werden.

Der Bis-(2-cyanethyl)ether kann in reiner, destillierter Form oder als Lösung des Ethers in einem inerten Lösungsmittel eingesetzt werden, bevorzugt werden Reaktionsausträge, die neben Bis-(2-cyanethyl)ether III in einer Konzentration von 10 bis 90 Gew.-% noch Wasser und Acrylnitril, Ethylencyanhydrin II sowie andere Nebenprodukte enthalten. Die Umsetzung kann auch in sich unter den Reaktionsbedingungen inert verhaltenden Lösungsmitteln durchgeführt werden.

Als inerte organische Lösungsmittel eignen sich z.B. Ether wie z.B. Dioxan, Tetrahydrofuran, Dimethylformamid oder Ethylenglykoldimethylether.

Der Reaktionsaustrag kann auf die übliche Art und Weise in seine Komponenten aufgetrennt werden. Da der Reaktionsaustrag nur geringe Anteile an Verunreinigungen enthält, kann er ohne weitere Reinigung weiterverarbeitet werden. Beispielsweise ist es möglich, die im Reaktionsaustrag enthaltenen Verbindungen Ethylencyanhydrin und 3-Aminopropionitril gemeinsam in einer Hydrierstufe zu den entsprechenden Aminen zu hydrieren.

Ethylencyanhydrin II und 3-Aminopropionitril I sind wichtige Zwischenprodukte für die chemische Industrie. 3-Aminopropionitril I eignet sich als Zwischenprodukt für die Herstellung von β-Alanin (Vorprodukt für Calciumpantothenat - DE-A-22 23 236), sowie von Propylendiamin, das in Pharmazeutika, Polyamiden und Holzschutzmitteln (DE-A-32 48 326, DE-A-20 04 405) eingesetzt wird.

Ethylencyanhydrin II findet vielseitige Verwendung als Zwischenprodukt für die Herstellung von Farbstoffen, Pflanzenschutzmitteln, Pharmaka und Kunststoffen.

### Beispiele

### Beispiel 1

In einem 1 l-Rührreaktor wurden 517 g Acrylnitril und 176 g Wasser in Gegenwart von 7,2 g Benzyl-dimethyl-(hydroxyethyl)ammoniumhydroxid bei 80°C innerhalb von 3 h bei 80°C miteinander umgesetzt.

50 ml/h dieses Reaktionsgemisches wurden mit 500 ml/h Ammoniak versetzt und das Gemisch wurde kontinuierlich durch einen ölbeheizten Rohrreaktor mit einer Länge von 1700 mm und einem Durchmesser von 30 mm, gefüllt mit Keramik-Ringen, bei 90°C und einem Gesamtdruck von 200 bar gepumpt. Das Reaktionsgemisch wurde in Gegenwart von Wasserstoff kontinuierlich an einem Cobalt-Katalysator bei 100°C hydriert und nach Entfernen von Ammoniak durch Destillation gaschromatographisch analysiert. Das Gemisch wies folgende Zusammensetzung auf:
10,6 % Wasser
33,9 % 1,3-Propylendiamin
35,9 % 3-Aminopropanol
8,6 % Bis-(3-aminopropyl)ether
2,9 % Dipropylentriamin
8,0 % Sonstige

### Beispiel 2

Durch einen ölbeheizten Rohrreaktor (Volumen 18,5 ml; 2,6 m Länge) wurden bei einer Temperatur von 100°C und einem Druck von 200 bar 70 ml/h Acrylnitril und 20 ml/h einer Lösung aus 2 Gew.-% Benzyl-dimethyl-(hydroxyethyl)ammoniumhydroxid in Wasser gepumpt.

Zu dem Reaktionsgemisch wurden kontinuierlich 500 ml/h Ammoniak zudosiert und das Gemisch wurde in einem zweiten ölbeheizten Rohrreaktor mit einem Volumen von 150 ml, gefüllt mit 150 ml V2A-Ringen, bei 120°C und einem Gesamtdruck von 200 bar umgesetzt. Nach destillativem Entfernen von Ammoniak wies der Reaktionsaustrag nach gaschromatographischer Analyse folgende Zusammensetzung auf:
17,1 % Wasser
38,5 % 3-Aminopropionitril
31,9 % Ethylencyanhydrin
0,3 % Bis-(2-cyanethyl)ether
6,5 % Bis-(2-cyanethyl)amin
0,2 % Acrylamid
4,8 % Sonstige

### Beispiel 3

In einem Rührreaktor wurde eine Mischung aus 48,6 g Acrylnitril und 15 g einer 0,1 N wäßrigen Lithiumhydroxidlösung 4 h bei 65°C gerührt. Nach Abtrennen der wäßrigen Phase wurden 45 ml/h des so erhaltenen Reaktionsgemischs zusammen mit 500 ml/h Ammoniak kontinuierlich in einen ölbeheizten Rohrreaktor mit einem Volumen von 150 ml, gefüllt mit 150 ml V_{A}-Ringen, bei 120°C und einem Gesamtdruck von 200 bar gepumpt. Nach Abdestillieren von Ammoniak wies der Reaktionsaustrag nach gaschromatographischer Analyse folgende Zusammensetzung auf:
11,4 % Wasser
37,9 % 3-Aminopropionitril
37,3 % Ethylencyanhydrin
- % Bis-(2-cyanethyl)ether
5,4 % Bis-(2-cyanethyl)amin
2,2 % Acrylamid
4,9 % Sonstige

### Beispiel 4

Durch einen ölbeheizten Rohrreaktor (Volumen 176 ml; 14 m Länge) wurden bei einer Temperatur von 70°C und einem Druck von 30 bar 135 ml/h Acrylnitril und 45 ml/h einer wäßrigen 0,1 N-Lösung von Lithiumhydroxid gepumpt. Das Reaktionsgemisch wurde mit 1000 ml/h Ammoniak versetzt und kontinuierlich in einen zweiten ölbeheizten Rohrreaktor mit einem Volumen von 290 ml, gefüllt mit 290 ml V2A-Ringen, bei 120°C und einem Gesamtdruck von 200 bar eindosiert und in einem dritten Rohrreaktor an einem Cobalt-Katalysator bei 120°C kontinuierlich hydriert. Nach Entfernen von Ammoniak wies der Reaktionsaustrag nach gaschromatographischer Analyse folgende Zusammensetzung auf:
13,3 % Wasser
46,7 % 1,3-Propylendiamin
15,6 % 3-Aminopropanol
0,4 % Bis-(3-aminopropyl)ether
10,6 % Sonstige
13,4 % Dipropylentriamin

### Beispiel 5

Durch einen ölbeheizten Rohrreaktor mit einem Volumen von 100 ml, gefüllt mit 100 ml Quarzkugeln, wurden bei 140°C und einem Gesamtdruck von 200 bar 122,7 ml/h Ammoniak und 21,4 ml/h destillativ gereinigter Bis-(2-cyanethyl)ether (Reinheit 90 %) eingespeist. Nach Entfernen von Ammoniak durch Destillation wies der Reaktionsaustrag nach gaschromatographischer Analyse folgende Zusammensetzung auf:
7,7 % Wasser
30,7 % 3-Aminopropionitril
38,5 % Ethylencyanhydrin
17,4 % Bis-(2-cyanethyl)ether
1,7 % Bis-(2-cyanethyl)amin
4,0 % Sonstige.

### Beispiel 6

Durch einen ölbeheizten Rohrreaktor (Volumen 90 ml; 7,16 m Länge) wurden bei einer Temperatur von 70°C und einem Druck von 80 bar 120 ml/h Acrylnitril und 45 ml/h einer wäßrigen 0,2 N-Lösung von Natriumhydroxid gepumpt. 80 g des Reaktionsgemisches wurden mit 1000 ml Ammoniak und 30 g Raney-Cobalt 1 h bei 30°C im Autoklaven gerührt. Danach wurden 150 bar Wasserstoff aufgedrückt. Nach 10stündigem Rühren bei 70°C und Entfernen des Ammoniaks durch Destillation wies der Reaktionsaustrag nach gaschromatographischer Analyse folgende Zusammensetzung auf:
19,5 % Wasser
39,5 % 1,3-Propylendiamin
29,2 % 3-Aminopropanol
7,1 % Dipropylentriamin
4,7 % Sonstige.

## Patentansprüche

1. Verfahren zur Herstellung von Gemischen aus 3-Aminopropionitril der Formel I
H₂N-CH₂-CH₂-CN (I)
und Ethylencyanhydrin der Formel II,
HO-CH₂-CH₂-CN (II)
dadurch gekennzeichnet, daß man Bis-(2-cyanethyl)ether der Formel III
NC-CH₂-CH₂-O-CH₂-CH₂-CN (III)
bei Temperaturen von 50 bis 200°C und Drücken von 0,1 bis 500 bar mit Ammoniak umsetzt.

2. Verfahren zur Herstellung von Gemischen aus 3-Aminopropionitril I und Ethylencyanhydrin II nach Anspruch 1, dadurch gekennzeichnet, daß man die Umsetzung bei 90 bis 150°C durchführt.

3. Verfahren zur Herstellung von Gemischen aus 3-Aminopropionitril I und Ethylencyanhydrin II nach Anspruch 1, dadurch gekennzeichnet, daß man die Umsetzung unter einem Druck von 1 bis 500 bar durchführt.

4. Verfahren zur Herstellung von Gemischen aus 3-Aminopropionitril I und Ethylencyanhydrin II nach Anspruch 1, dadurch gekennzeichnet, daß man die Umsetzung unter einem Druck von 20 bis 300 bar durchführt.

5. Verfahren zur Herstellung von Gemischen aus 3-Aminopropionitril I und Ethylencyanhydrin II nach Anspruch 1, dadurch gekennzeichnet, daß man die Umsetzung kontinuierlich durchführt.

6. Verfahren zur Herstellung von Gemischen aus 3-Aminopropionitril I und Ethylencyanhydrin II nach Anspruch 1, dadurch gekennzeichnet, daß man die Umsetzung in Rohrreaktoren durchführt.

7. Verfahren zur Herstellung von Gemischen aus 3-Aminopropionitril I und Ethylencyanhydrin II nach Anspruch 1, dadurch gekennzeichnet, daß man Ammoniak und Bis-(2-cyanethyl)ether III im Molverhältnis von 1 : 1 bis 100 : 1 einsetzt.

8. Verfahren zur Herstellung von Gemischen aus 3-Aminopropionitril I und Ethylencyanhydrin II nach Anspruch 1, dadurch gekennzeichnet, daß man Ammoniak und Bis-(2-cyanethyl)ether III im Molverhältnis von 10 : 1 bis 50 : 1 einsetzt.
